# EUROPÄISCHE PATENTSCHRIFT

(11) **EP 2 656 767 B1**
(45) Veröffentlichungstag und Bekanntmachung des Hinweises auf die Patenterteilung: **08.06.2016**
(21) Anmeldenummer: 12401072.9
(22) Anmeldetag: 27.04.2012
(51) Int. Cl.: A61B 90/80

(54) **Reinigungs- und Desinfektionsautomat**
Cleaning and disinfection machine
Automate de nettoyage et de désinfection

(43) Veröffentlichungstag der Anmeldung: 30.10.2013
(73) Patentinhaber: Miele & Cie. KG, 33332 Gütersloh (DE)
(72) Erfinder: Schröder, Frank, 32139 Spenge (DE); Thorman, Franz, 33649 Bielefeld (DE)

(56) Entgegenhaltungen:
- WO-A1-2009/016111
- DE-A1- 2 919 140
- DE-A1- 3 413 386

## Beschreibung

Die Erfindung betrifft einen Reinigungs- und Desinfektionsautomat insbesondere für medizinische Instrumente und Geräte, mit einem einen Spülraum bereitstellenden Spülbehälter und einer im Spülraum angeordneten Sprüheinrichtung, wobei die Sprüheinrichtung eine Mehrzahl von um eine gemeinsame Drehachse verdrehbar ausgebildeten Sprüharmen aufweist.

Reinigungs- und Desinfektionsautomaten der vorbeschriebenen Art sind aus dem Stand der Technik an sich gut bekannt, beispielsweise aus der DE 10 2009 009 768 A1 und der DE 10 2006 009 787 A1.

Vorbekannte Reinigungs- und Desinfektionsautomaten, nachfolgend auch Spülautomaten genannt, verfügen über einen Spülbehälter, der einen Spülraum bereitstellt. Im bestimmungsgemäßen Verwendungsfall dient der Spülraum der Aufnahme von zu reinigendem und/oder zu desinfizierendem Spülgut, bei dem es sich beispielsweise um medizinische Instrumente und Geräte handeln kann. Der Spülraum ist über eine Beschickungsöffnung des Spülbehälters zugänglich, die mittels einer verschwenkbar am Spülbehälter angeordneten Tür fluiddicht verschließbar ist.

Typischerweise kommt im bestimmungsgemäßen Verwendungsfall zur Spülgutreinigung ein Spülkorb zum Einsatz. Dieser wird mit dem zu reinigenden Spülgut bestückt und anschließend verwenderseitig durch die Beschickungsöffnung hindurch in den Spülraum eingeführt. Nach erfolgreich durchgeführter Spülung, das heißt Reinigung und/oder Desinfektion des Spülguts kann der Spülkorb zusammen mit den davon aufgenommenen Spülgütern verwenderseitig dem Spülraum durch die Beschickungsöffnung wieder entnommen werden.

Innerhalb des Spülraums ist eine Sprüheinrichtung angeordnet. Diese dient der Beschickung von Spülgut mit Spülflotte. Die Sprüheinrichtung verfügt über eine Mehrzahl von um eine gemeinsame Drechachse verdrehbar ausgebildeten Sprüharmen. Es kann dabei zwischen automatenseitigen Sprüharmen einerseits und korbseitigen Sprüharmen andererseits unterschieden werden. Dabei betreffen "automatenseitige Sprüharme" solche Sprüharme, die im Spülraum des Automaten installiert sind. In der Regel sind zwei solcher Sprüharme vorgesehen, wobei der eine deckenseitig und der andere bodenseitig des Spülraums ausgebildet ist. "Korbseitige Sprüharme" betreffen indes solche Sprüharme, die am Spülkorb angeordnet sind, die als zusammen mit dem Spülkorb verwenderseitig dem Spülraum entnehmbar sind. Zum Anschluss korbseitiger Sprüharme an ein Spülflottenzuführsystem sind im Spülraum entsprechend ausgebildete Ankopplungsstellen vorgesehen, wie sie beispielsweise aus der DE 10 2007 003 894 A1 bekannt sind.

Die Sprüheinrichtung kann je nach Ausgestaltung des Reinigungs- und Desinfektionsautomaten nicht nur der Abgabe von Spülflotte dienen. Es sind Ausgestaltungsformen bekannt geworden, wonach die Sprüheinrichtung auch dazu dient, das Spülgut nach erfolgter Reinigung und/oder Desinfektion zu trocknen, zu welchem Zweck Trocknungsluft in den Spülraum über die Sprüheinrichtung eingeleitet wird. In diesem Fall dient die Sprüheinrichtung einerseits dazu, während des eigentlichen Reinigungs- und/oder Desinfektionsvorganges Spülflotte auf das zu reinigende und/oder zu desinfizierende Spülgut abzugeben sowie andererseits dazu, nach Abschluss eines solchen Reinigungs- und/oder Desinfektionsvorganges das Spülgut mit Trocknungsluft zu beaufschlagen.

Zur Versorgung der Sprüheinrichtung entweder mit Spülflotte oder mit Trocknungsluft sind insbesondere bei Großgeräten jeweils Speiseleitungen vorgesehen, die über außerhalb des Spülraums ausgebildete Verteileinrichtungen strömungstechnisch miteinander koppelbar beziehungsweise voneinander entkoppelbar sind.

Zum Zwecke der Umwälzung von Spülflotte kommt eine Umwälzpumpe zum Einsatz. Über diese wird im Spülraum befindliche Spülflotte angesaugt und über entsprechende Versorgungsrohre den Sprüharmen oder sonstigen Fluidabgabeeinrichtungen der Spüleinrichtung zugeführt. Diese Versorgungsrohre sind zur Maximierung der Größe des nutzbaren Spülraums außerhalb des Spülraums verlegt, wie sich dies beispielsweise auch aus den schon vorstehend genannten Druckschriften DE 10 2009 009 778 A1 und DE 10 2006 009 787 A1 ergibt. Die Anordnung der Versorgungsrohre außerhalb des Spülraums ist darüber hinaus mit Blick auf die schon vorgenannte Ankopplung von korbseitigen Sprüharmen an ein Fluidzuführungssystem von Vorteil.

Aus der DE 2919140 A1 ist darüber hinaus ein Reinigungs- und Desinfektionsautomat bekannt, welcher ein sich entlang der Drehachse der Sprüharme erstreckendes Versorgungsrohr zur Versorgung der Sprüharme mit Spülflotte aufweist, wobei das Versorgungsrohr einen im Bodenbereich des Spülraums fest installierten Verbindungsrohrstutzen und mehrere spülkorbseitige Anschlussrohrstutzen aufweist.

Obgleich sich die vorbeschriebene Konstruktion im alltäglichen Praxiseinsatz bewährt hat, besteht Verbesserungsbedarf, insbesondere mit Blick auf eine noch weiter vereinfachte Handhabung.

Es ist deshalb ausgehend von dem Vorbeschriebenen die Aufgabe der Erfindung, einen neuartigen Reinigungs- und Desinfektionsautomaten vorzuschlagen, der bei gleichzeitiger Verbesserung der Betriebssicherheit eine vereinfachte Handhabung gestattet.

Zur Lösung dieser Aufgabe wird mit der Erfindung ein Reinigungs- und Desinfektionsautomat mit den Merkmalen von Anspruch 1 vorgeschlagen.

Bei der aus dem Stand der Technik bekannten, gängigen Konstruktion kommen Versorgungsleitungen zum Einsatz, die außerhalb des Spülraums angeordnet sind, nicht zuletzt deshalb, um Ankopplungsstellen schaffen zu können, die der Ankopplung der Versorgungsleitungen an korbseitige Sprüharme dienen. Obgleich sich diese Ausgestaltung im alltäglichen Praxiseinsatz bewährt hat, ist sie insofern von Nachteil, als dass sie leckageanfällig ist.

Die erfindungsgemäße Ausgestaltung schafft hier Abhilfe, und es wird mit ihr im Unterschied zum Stand der Technik ein völlig anderer Lösungsweg beschritten.

Nach der erfindungsgemäßen Ausgestaltung kommt ein zentrales Versorgungsrohr zum Einsatz. Dieses ist nicht außerhalb, sondern innerhalb der Spülkammer ausgebildet. Undichtigkeiten und Leckageprobleme sind somit auf ein Minimum reduziert.

Mit der Anordnung des Versorgungsrohrs innerhalb der Spülraums begegnet die Erfindung einem in der Fachwelt vorherrschenden Vorurteil. Es ist seitens der Fachwelt bislang die Auffassung vertreten worden, Versorgungsleitungen und -rohre außerhalb des Spülraums zu verlegen, um so die nutzbare Größe des Spülraums zu maximieren. Tatsächlich bewirkt aber die nunmehr vorgeschlagene Anordnung des Versorgungsrohrs innerhalb des Spülraums eine Kapazitätserweiterung. Dies deshalb, weil der aufgrund des vollständigen Verzichts auf außerhalb des Spülraums verlegte Versorgungsleitungen frei werdende Bauraum für eine Spülraumvergrößerung genutzt werden kann, und dies bei gleichbleibenden Grundabmessungen des Automatens. Die insgesamt erreichte Spülraumvergrößerung wiegt den für die Verlegung des Versorgungsrohrs innerhalb des Spülraums benötigten Platzbedarf auf, wobei insgesamt eine Kapazitätserweiterung erreicht wird.

Das mit der Erfindung vorgeschlagene Versorgungsrohr erstreckt sich entlang der Drehachse der Sprüharme der Sprüheinrichtung. Es ist einendseitig an eine Umwälzpumpe angeschlossen, so dass im Betriebsfall von der Umwälzpumpe geförderte Spülflotte direkt in das Versorgungsrohr eingeleitet wird, von wo aus dann eine Beschickung der mit dem Versorgungsrohr gekoppelten Sprüharme stattfindet.

Dabei ist das Versorgungsrohr in drei Abschnitte unterteilt, und es verfügt über einen ersten, einen zweiten und einen dritten Abschnitt. Zwei der Abschnitte sind automatenseitig ausgebildet. Der dritte Abschnitt des Versorgungsrohrs ist vom Spülkorb aufgenommen, wobei er axial verschieblich gelagert ist. Im bestimmungsgemäßen Verwendungsfall ist dieser spülkorbseitige Abschnitt des Versorgungsrohrs zwischen die beiden automatenseitigen Abschnitte fluiddicht eingebracht, so dass von der Umwälzpumpe umgewälzte Spülflotte durch sämtliche Abschnitte des Versorgungsrohrs hindurch zu den einzelnen Sprüharmen strömen kann.

Nach erfolgreich abgeschlossenen Spülprogramm können die Abschnitte des Versorgungsrohrs voneinander getrennt werden, was es gestattet, den Spülkorb samt dem davon getragenen Spülgut dem Spülraum zwecks Entladung entnehmen zu können. Der spülkorbseitige Abschnitt des Versorgungsrohrs dient insofern als strömungstechnisches Koppelglied zwischen den beiden automatenseitigen Abschnitten. Auf eine Ankopplung korbseitiger Sprüharme an eine außerhalb des Spülraums verlegte Anschlussleitung kann dank der erfindungsgemäßen Ausgestaltung vollends verzichtet werden, so dass insbesondere Leckageprobleme und/oder sonstige Undichtigkeiten mit der erfindungsgemäßen Ausgestaltung vollends vermieden werden können.

Die Aufnahme des mittleren Abschnitts des Versorgungsrohrs durch den Spülkorb erfolgt bevorzugterweise schwimmend. "Schwimmend" meint dabei, dass eine axiale Verschiebung des Versorgungsrohrabschnittes relativ gegenüber dem Spülkorb gestattet ist und dass darüber hinaus ein Toleranzausgleich in radialer Richtung möglich ist. Eine solche Lagerung kann beispielsweise dadurch erreicht werden, dass der Versorgungsrohrabschnitt der Gewichtskraft folgend auf entsprechenden Widerlagern des Spülkorbs aufliegt. Sobald der Spülkorb in den Spülraum verbracht und eine fluiddichte Verbindung zwischen den einzelnen Abschnitten des Versorgungsrohrs ausgebildet ist, wird der spülkorbseitige Abschnitt des Versorgungsrohrs nicht mehr vom Spülkorb beziehungsweise dem vom Spülkorb bereitgestellten Widerlagen, sondern von den automatenseitigen Abschnitten des Versorgungsrohrs abgestützt.

Einer der beiden automatenseitigen Abschnitte des Versorgungsrohrs ist axial verschieblich gelagert. Bevorzugterweise ist diese axiale Verschieblichkeit bezüglich des bodenseitigen Versorgungsrohrschnittes möglich.

Die axiale Verschieblichkeit eines der beiden automatenseitigen Versorgungsrohrabschnitte dient dazu, eine fluiddichte Verbindung der einzelnen Rohrabschnitte ausbilden beziehungsweise lösen zu können. In der Ausgangsposition ist der Abstand zwischen den beiden automatenseitigen Abschnitten des Versorgungsrohrs größer als die Längserstreckung des korbseitigen Rohrabschnitts. Auf diese Weise ist es gestattet, den Spülkorb zusammen mit dem davon aufgenommenen Versorgungsrohrabschnitt in bestimmungsgemäßer Weise in den Spülraum des Automaten einzubringen. Der korbseitige Rohrabschnitt kann in dieser Position zwischen die beiden automatenseitigen Rohrabschnitte verbracht werden. Alsdann ist der axial verschiebliche automatenseitige Rohrabschnitt in Längsrichtung, das heißt axial zu verschieben. In der Konsequenz wird der Abstand zwischen den beiden automatenseitigen Rohrabschnitten verkleinert, was zu einer Einklemmung des korbseitigen Rohrabschnittes zwischen den beiden automatenseitigen Rohrabschnitten führt. Im Ergebnis stellt sich eine fluiddichte Verbindung der einzelnen Abschnitte des Versorgungsrohrs ein, so dass im bestimmungsgemäßen Verwendungsfall eine Beschickung der Sprüheinrichtung über das Versorgungsrohr mit Spülflotte erfolgen kann.

Zum Zwecke der axialen Verschiebung des einen automatenseitigen Rohrabschnittes ist bevorzugterweise ein Hubzylinder vorgesehen, der mit diesem Abschnitt zusammenwirkt. Dabei kann als Hubzylinder vorzugsweise ein pneumatisch arbeitender Hubzylinder vorgesehen sein.

Der spülkorbseitige Abschnitt des Versorgungsrohrs ist endseitig bevorzugterweise mit konischen Erweiterungen ausgerüstet. Die automatenseitigen Abschnitte des Versorgungsrohrs sind zu diesen konischen Erweiterungen korrespondierend ausgebildet und verfügen über entsprechende Anschlussstutzen. In ihrem Zusammenwirken bilden die Anschlussstutzen und die konischen Erweiterungen eine fluiddichte Verbindung aus.

Die automatenseitigen Abschnitte des Versorgungsrohrs sind kugelgelagert im Spülraum angeordnet. Eine Verdrehbewegung der Rohrabschnitte und damit der davon getragenen Sprüharme um die Drehachse ist so gestattet.

Die Verbindung der einzelnen Rohrabschnitte über die zugehörigen Anschlussstutzen und konischen Erweiterungen unterliegen im Vergleich zu der Kugellagerung der automatenseitigen Rohrabschnitte einer größeren Reibung. In der Konsequenz ergibt sich über eine kraftübertragende Verbindung der einzelnen Rohrabschnitte zueinander, so dass über einen Antrieb allein des oberen oder unteren automatenseitigen Rohrabschnittes ein Antrieb sämtlicher Rohrabschnitte und der damit verbundenen Sprüharme erreicht ist.

Der spülkorbseitige Abschnitt des Versorgungsrohrs kann in einzelne fluiddicht miteinander verbindbare Segmente unterteilt sein. Diese Ausgestaltung ist dann bevorzugt, wenn anstelle eines einzelnen Spülkorbes zu einem Spülkorb zusammengesetzte Spülkorbelemente oder -fächer eingesetzt werden. In diesem Fall verfügt ein jedes Spülkorbfach über ein eigenes Versorgungsrohrsegment, wobei die Segmente aller miteinander zu einem Spülkorb kombinierten Spülkorbfächer den spülkorbseitigen Abschnitt des Versorgungsrohrs ausbilden.

Gemäß einem weiteren Merkmal ist vorgesehen, dass an das Versorgungsrohr einendseitig eine Umwälzpumpe und anderendseitig ein Gebläse angeschlossen ist. Dies gestattet es, die Sprüheinrichtung entweder mit Spülflotte über die Umwälzpumpe oder mit Trockenluft über das Gebläse zu beschicken.

Der Spülraum mündet in einen Sammeltopf ein. Dabei ist es bevorzugt, dass der Sammeltopf der Umwälzpumpe als Pumpengehäuse dient, zu welchem Zweck der Sammeltopf und das Pumpengehäuse als ein Bauteil ausgebildet sind.

Die Ausgestaltung des Sammeltopfes als Pumpengehäuse ergibt in der Konsequenz, dass die Umwälzpumpe im Vergleich zu den aus dem Stand der Technik bekannten Konstruktionen um 90° verdreht ausgerichtet ist, das heißt das Pumpenrad der Umwälzpumpe und die im Spülraum angeordneten Sprüharme der Sprüheinrichtung um ein und dieselbe Drehachse drehen.

Das Pumpenrad der Umwälzpumpe wirkt mit einem fest- beziehungsweise stillstehenden Leitrad zusammen. Die im Betriebsfall am Umfang des Pumpenrades austretende Wassermenge wird durch dieses Leitrad aufgefangen und zur Drehachse hin umgelenkt, so dass das Wasser ohne weitere Umwege und Umlenkungen in Richtung der Drehachse in das im Innenraum des Automaten angeordnete Versorgungsrohr eingeleitet werden kann.

Die das Pumpenrad antreibende Pumpenwelle ist bevorzugterweise als Hohlwelle ausgebildet. Dies gestattet es, die Sprüharme über eine Antriebswelle anzutreiben, die die Hohlwelle durchragt, womit eine sehr kompakte Ausgestaltung erreicht ist. Dabei hat ein gesonderter Antrieb der Sprüharme den Vorteil, dass sich über eine Rückmeldung des elektrischen Antriebes Aussagen über Drehzahl und Blockierungsfreiheit der Sprüharme ableiten lassen, was wiederum in einer Steuerung ausgewertet werden kann. Ein vorbestimmter und nachsteuerbarer Drehbetrieb der Sprüharme ist damit gestattet.

Die Sprüheinrichtung kann auch zur Beaufschlagung des Spülguts mit Trocknungsluft genutzt werden. Zu diesem Zweck ist das zentral im Spülraum angeordnete Versorgungsrohr anderendseitig an ein Gebläse angekoppelt. Die vom Gebläse erzeugte Trocknungsluft wird in das Versorgungsrohr eingeleitet, von wo aus die einzelnen Sprüharme und/oder sonstigen Fluidabgabeeinrichtungen der Sprüheinrichtung mit Trocknungsluft versorgt werden.

Die erfindungsgemäße Konstruktion erbringt gegenüber dem Stand der Technik insgesamt insbesondere folgende Vorteile:
- Durch die Anordnung des Versorgungsrohrs ausschließlich im Spülraum ist die Länge der Wasserwege und die Anzahl der Umlenkungen deutlich reduziert, wobei eine Minimierung der Strömungsverluste erreicht ist. Gemäß der vorgeschlagenen Konstruktion fördert die Umwälzpumpe direkt in Richtung der zentralen Spülflottenführung in den Spülraum hinein.
- Gegenüber dem Stand der Technik ist eine deutliche Reduzierung des Volumens für die Wasserführung erreicht, womit die in den Führungswegen gebundene Wassermenge mit dem Vorteil reduziert ist, dass bei bestimmungsgemäßer Verwendung eine gegenüber dem Stand der Technik verringerte Menge an Frischwasser erforderlich ist.
- Die Anzahl möglicher Leckagestellen ist deutlich verringert, was die Betriebssicherheit insgesamt erhöht.
- Die Anzahl der eingesetzten Bauteil und Baukomponenten ist deutlich reduziert, was nicht zuletzt eine Kostenreduktion in der Herstellung und Wartung erbringt.
- Auf außerhalb des Spülraums verlegte Versorgungsleitungen wird vollständig verzichtet. Dies gestattet es, den hierdurch frei werdenden Bauraum für eine Spülraumvergrößerung zu nutzen. Im Ergebnis erbringt dies eine Kapazitätserweiterung, was ebenfalls dazu beiträgt, Ressourcen schonen zu können.
- Das Spülraumunterteil ist im Unterschied zum Stand der Technik fertigungstechnisch deutlich vereinfacht, da es als Prägeteil und nicht mehr als Tiefziehteil mit angebrachtem Schweißteil ausgebildet werden kann, was die Herstellung insgesamt vereinfacht und kostengünstiger gestaltet.
- Der Restwasser- beziehungsweise Spülflottenablauf ist erheblich verbessert, da auf verzweigte, externe Wasserwege konstruktiv verzichtet ist. Eine Entleerung solcher Wasserwege im Unterschied zum Stand der Technik also nicht weiter erforderlich ist.
- Es kann ferner auf Bypassspülungen für entlegene Ablaufelemente und -schläuche verzichtete werden, da derartige entlegene Ablaufelemente und-schläuche bei der erfindungsgemäßen Konstruktion nicht vorhanden sind.

Die Unterteilung des Versorgungsrohrs in unterschiedliche Abschnitte macht es möglich, nach dem Baukastenprinzip miteinander kombinierte Spülkörbe einzusetzen, wobei ein Anschluss der einzelnen Abschnitte zum Zwecke der Spülflottenversorgung nicht über außerhalb des Spülraums verlaufende Versorgungsrohre oder -leitungen erfolgt, womit die Leckageproblematik verringert ist.
- Der in einfacher Weise realisierbare motorische Antrieb der Sprüharme ermöglicht es, für das jeweils zu reinigende Spülgut optimierte Drehzahlen wählen zu können, Drehrichtungswechseln, Step-Drehungen und/oder dergleichen programmtechnisch vorsehen zu können. Dabei ist die Sprüharmdrehzahl nicht mehr abhängig vom Spüldruck.
- Die Korbankopplung erlaubt, Energie einzusparen, da weniger Wasser gebunden wird, und dies bei gleichzeitiger Steigerung der Reinigungsintensität. Verkürzte Durchlaufzeiten der Spülprogramme sind in vorteilhafter Weise die Folge.

Es ergibt sich damit alles in allem eine gegenüber dem Stand der Technik deutliche vereinfachte Handhabung.

Weitere Vorteile und Merkmale der Erfindung ergeben sich aus der nachfolgenden Beschreibung anhand der Figuren.

Dabei zeigen
- Fig. 1: in einer schematischen Ansicht einen Reinigungs- und Desinfektionsautomaten nach der Erfindung;
- Fig. 2: in einer schematischen Darstellung insbesondere den Spülbehälter eines erfindungsgemäßen Reinigungs- und Desinfektionsautomaten;
- Fig. 3: in einer ersten Ansicht das Versorgungsrohr nach der Erfindung in entkoppelten Zustand;
- Fig. 4: in einer zweiten Ansicht das Versorgungsrohr nach Fig. 3;
- Fig. 5: in einer weiteren Ansicht das Versorgungsrohr nach Fig. 3, allerdings im verkoppelten Zustand und
- Fig. 6: in einer weiteren Ansicht das Versorgungsrohr gemäß Fig. 5.

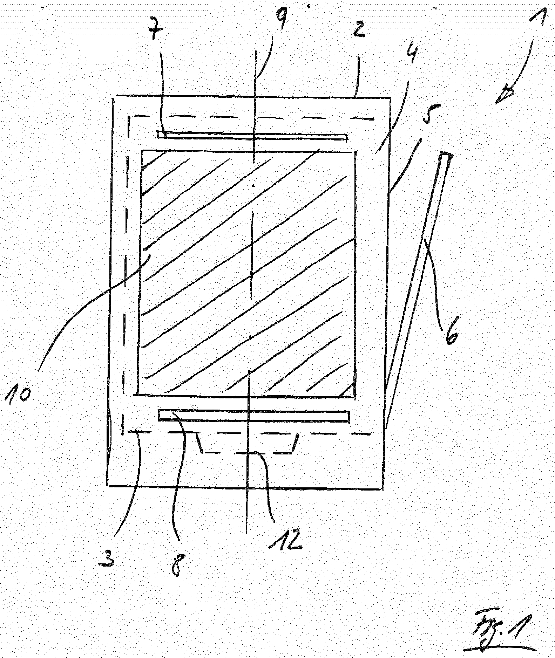

Fig. 1 lässt in schematischer Seitenansicht einen Reinigungs- und Desinfektionsautomaten nach der Erfindung erkennen, im nachfolgenden Spülautomat 1 genannt.

Der Spülautomat 1 nach der Erfindung ist als Gewerbeautomat ausgelegt und dient insbesondere der Behandlung von medizinischen Instrumenten und Geräten, beispielsweise Glaskolben, Reagenzgläsern, Schläuchen und/oder dergleichen.

Der Spülautomat 1 verfügt über ein von einem Gestellt 11 getragenes Gehäuse 2. Innerhalb des Gehäuses 2 ist ein Spülbehälter 3 angeordnet, der einen Spülraum 4 bereitstellt. Der Spülraum 4 ist über eine Beschickungsöffnung 5 zugänglich, welche Beschickungsöffnung 5 mittels einer Spülraumtür 6 fluiddicht verschließbar ist.

Im bestimmungsgemäßen Verwendungsfall dient der Spülraum 4 der Aufnahme von zu reinigendem Spülgut, beispielsweise medizinischen Instrumenten und Geräten. Zur Positionierung des zu reinigenden Spülguts innerhalb des Spülraums 4 dient ein Spülkorb 10. Dieser kann verwenderseitig aus dem Spülraum 4 herausverfahren beziehungsweise in diesen eingebracht werden.

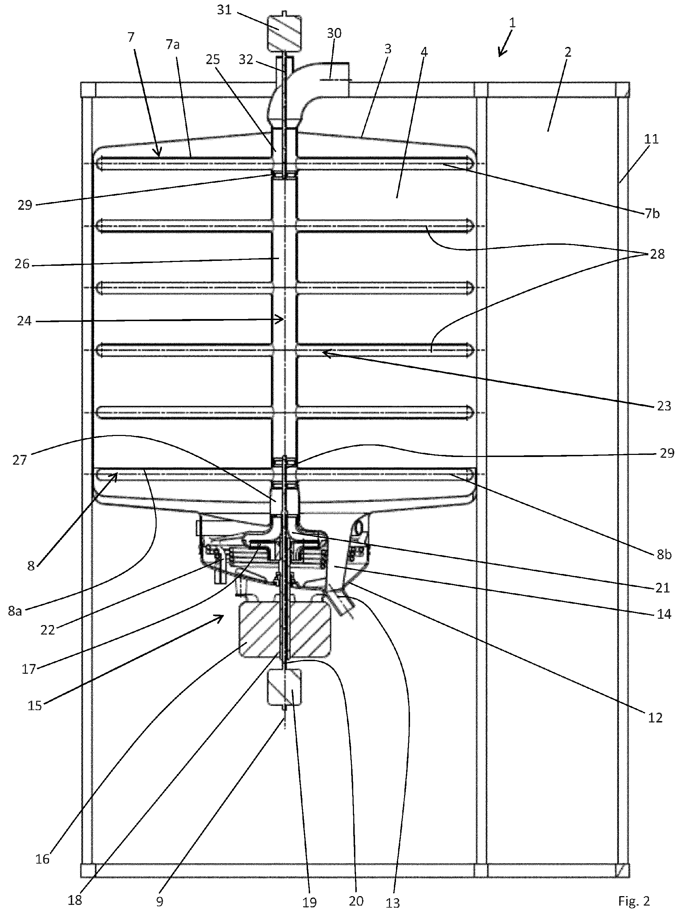

Zum Zwecke der Beschickung des Spülraums 4 mit Spülflotte oder mit Trocknungsluft kommt eine Sprüheinrichtung 23 zum Einsatz, wie sie sich insbesondere aus der Darstellung nach Fig. 2 ergibt. Die Sprüeinrichtung 23 verfügt einerseits über automatenseitige Sprüharme 7 und 8 sowie andererseits über korbseitige Sprüharme 28. Der automatenseitige Sprüharm 7 ist innerhalb des Spülraums 4 deckenseitig montiert und verfügt über zwei Sprüharmäste 7a und 7b. Der zweite automatenseitige Sprüharm 8 ist in gleicher Weise ausgebildet und verfügt über Sprüharmäste 8a und 8b. Dabei ist der Sprüharm 8 bodenseitig des Spülraums 4 ausgebildet. Beide Sprüharme 7 und 8 drehen um die gemeinsame Drehachse 9.

Der Spülraum 4 mündet bodenseitig in einen Sammeltopf 12 ein, wie dies insbesondere der schematischen Darstellung nach Fig. 1 entnommen werden kann.

Fig. 2 lässt den Aufbau des erfindungsgemäßen Spülautomaten 1 genauer erkennen. Wie dieser Darstellung zu entnehmen ist, ist der Spülbehälter 3 innerhalb des von dem Gestell 11 getragenen Gehäuses 2 angeordnet. Der Spülbehälter 3 geht bodenseitig in den Sammeltopf 12 über.

Zum Zwecke der Umwälzung der im bestimmungsgemäßen Verwendungsfall im Spülraum 4 befindlichen Spülflotte dient eine Umwälzpumpe 15. Diese saugt die sich im Sammeltopf 12 ansammelnde Spülflotte an und fördert diese zur Sprüheinrichtung 23, von wo aus dann eine Beschickung von Spülgut mit Spülflotte erfolgt.

Der Sammeltopf 12 dient gemäß dem gezeigten Ausführungsbeispiel der Umwälzpumpe 15 als Pumpengehäuse, zu welchem Zweck der Sammeltopf 12 und das Pumpengehäuse als ein Bauteil ausgebildet sind. Fig. 2 lässt diese Ausgestaltung gut erkennen.

Der vom Sammeltopf 12 beziehungsweise dem Pumpengehäuse bereitgestellte Volumenraum ist vom übrigen Spülraum 4 durch ein Flächensieb 14 getrennt. Mittels diesem Flächensieb 14 können grobe Verschmutzungen zurückgehalten werden. Dabei ist es bevorzugt, das Flächensieb 14 mit einem Reservoir 14a auszurüsten, mit welchem vom Flächensieb 14 zurückgehaltene Partikel, Verschmutzungen und/oder dergleichen Fremdstoffe während der Durchführung eines Spülprogramms gesammelt werden können.

Der Sammeltopf 12 ist mit einem Ablaufstutzen 13 ausgerüstet. Dieser ist an der tiefsten Stelle des Sammeltopfes 12 ausgebildet und dient nach erfolgreich durchlaufenem Spülprogramm der Wasser- beziehungsweise Spülflottenentleerung. Dabei ist es bevorzugt, das Reservoir 14a des Flächensiebs 14 direkt oberhalb des Ablaufstutzens 13 auszugestalten, wie dies in Fig. 2 gezeigt ist. Im Reservoir 14a angesammelte Partikel, Verschmutzungen und/oder dergleichen Fremdstoffe können so bei einer Restwasserentleerung direkt über den Ablaufstutzen 13 abgepumpt werden.

Die Umwälzpumpe 15 verfügt über einen außerhalb des Sammeltopfes 12 ausgebildeten Motor 16. Dieser treibt über eine Pumpenwelle 18 das im Sammeltopf 12 angeordnete Pumpenrad 17 an. Dieses wirkt mit einem fest-, das heißt stillstehenden Leitrad 21 zusammen. Die im bestimmungsgemäßen Betriebsfall am Umfang des Pumpenrades 17 austretende Wassermenge wird durch das Leitrad 21 aufgefangen und in Richtung der Drehachse 9 umgelenkt und mit Bezug auf die Zeichnungsebene nach Fig. 2 nach oben in das Verteil- oder Versorgungsrohr 24 eingeleitet, von wo aus dann eine Bedienung der Sprüharme 7, 8 und 28 stattfindet.

Im Sammeltopf 12 beziehungsweise im Pumpengehäuse der Umwälzpumpe 15 können ferner Elektro- und/oder Dampfheizkörper in Form einer Heizeinrichtung 22 vorgesehen sein. Damit dient der Sammeltopf 12 nicht nur der Funktion als Ansaugvolumen für die Umwälzpumpe 15, er wird auch für die Unterbringung entsprechender Heizeinrichtungen 22 genutzt.

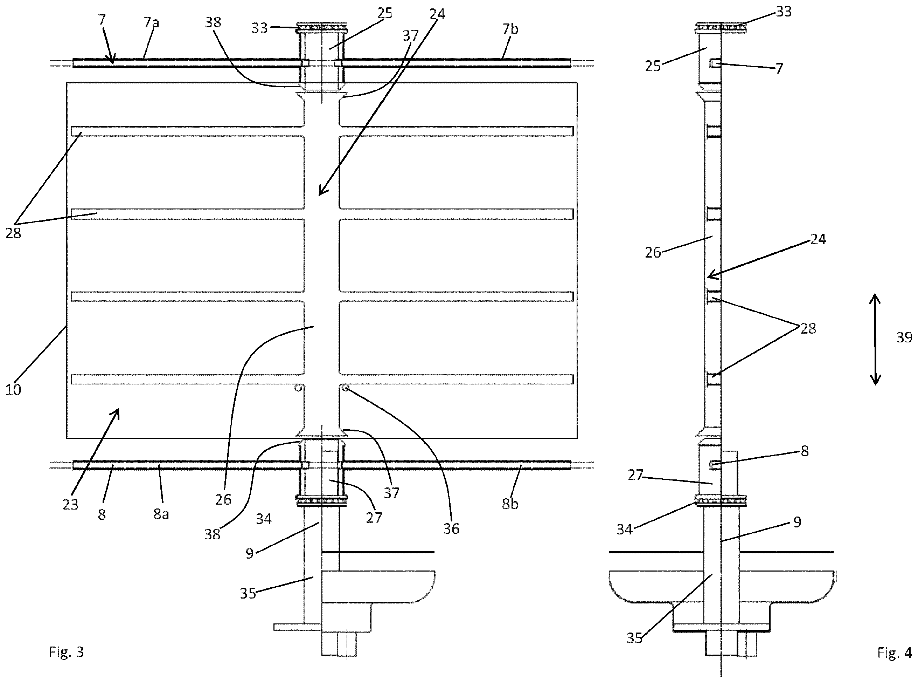

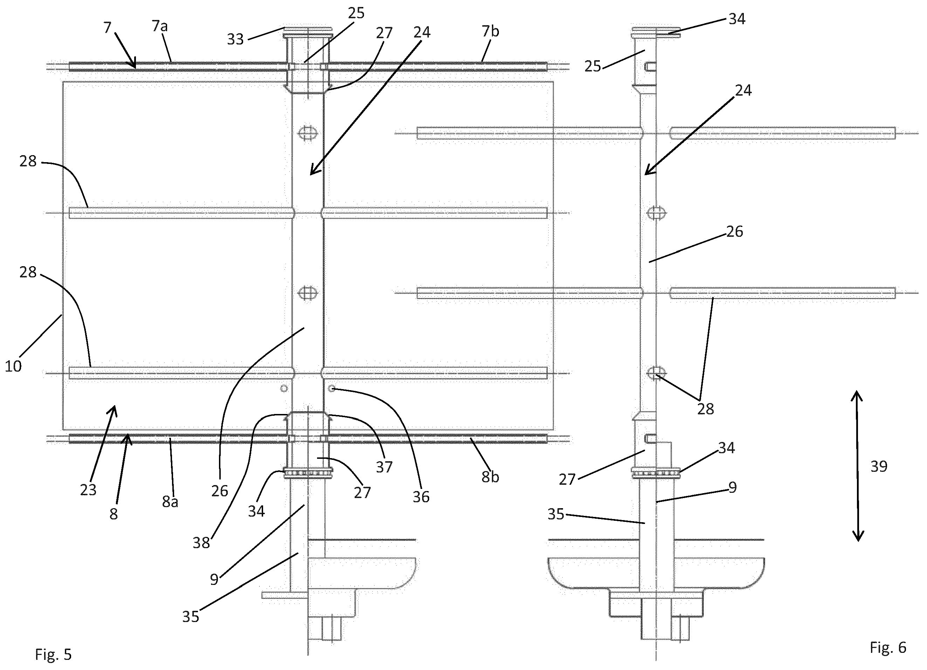

Das zur Versorgung der Sprüheinrichtung 23 mit Spülflotte vorgesehene Versorgungsrohr 24 verfügt über insgesamt drei Abschnitte 25, 26 und 27. Dabei dient der erste Abschnitt 25 als oberer Abschnitt, der mit dem deckenseitigen Sprüharm 7 gekoppelt ist. Der zweite Abschnitt 27 dient als unterer Abschnitt, der seinerseits mit dem bodenseitigen Sprüharm 8 gekoppelt ist. Der dritte Abschnitte 26 dient als mittlerer Abschnitt und ist korbseitig, das heißt als Bestandteil des dem Spülraum 4 entnehmbaren Spülkorbes 10 ausgebildet. Die Abschnitte 25, 26 und 27 sind im bestimmungsgemäßen Verwendungsfall fluiddicht miteinander verbunden, was dem gekoppelten Zustand gemäß der Fign. 5 und 6 entspricht. Die entkoppelte Stellung, das heißt die Trennung der Abschnitte 25, 26 und 27 voneinander ist in den Fign. 3 und 4 dargestellt.

Im einfachsten Ausführungsfall verfügt die Sprüheinrichtung 23 über die beiden automatenseitigen Sprüharme 7 und 8. Im bestimmungsgemäßen Verwendungsfalls ist der dritte, das heißt der mittlere Abschnitt 26 des Versorgungsrohrs 24 fluiddicht mit den beiden anderen Abschnitten 25 und 27 verbunden, so dass über die Umwälzpumpe 15 geförderte Spülflotte einerseits den bodenseitigen Sprüharm 8 und über das Versorgungsrohr 24 auch den deckenseitigen Sprüharm 7 erreicht.

Gemäß den in den Figuren gezeigten Ausführungsform sind weitere Sprüharme 28 vorgesehen, die als sprühkorbseitige Sprüharme 28 direkt an das Versorgungsrohr 24 angeschlossen sind. Dabei kann eine Anordnung der Sprüharme 28 auch versetzt zueinander erfolgen, wie dies beispielhaft in den Fign. 5 und 6 angedeutet ist. Im bestimmungsgemäßen Verwendungsfall findet über das Versorgungsrohr 24 mithin nicht nur eine Beschickung der Sprüharme 7 und 8, sondern auch eine Beschickung der mit dem Versorgungsrohr 24 gekoppelten Sprüharme 28 statt.

Zur Kuppelung des ersten Abschnitts 25 und des dritten Abschnitts 26 des Versorgungsrohrs 24 beziehungsweise des zweiten Abschnitts 27 und des dritten Abschnitts 26 des Versorgungsrohrs 24 sind jeweils Kupplungsstellen 29 vorgesehen, die für eine fluiddichte Verbindung sorgen. Dabei ist vorgesehen, dass der dritte, das heißt mittlere Abschnitt 26 des Versorgungsrohrs 24 sowohl einendseitig als auch anderendseitig über konische Erweiterungen 37 verfügt. Die zugehörigen automatenseitgen Abschnitte 25 und 26 verfügen über zu diesen konischen Erweiterungen 37 korrespondierend ausgebildete Anschlussstutzen 38, so dass im verkoppelten Zustand der einzelnen Abschnitte 25, 26 und 27 eine fluiddichte Verbindung zwischen den einzelnen Abschnitten ausgebildet ist.

Der mittlere Abschnitt 26, das heißt der spülkorbseitige Abschnitt des Versorgungsrohrs 24 ist mittels einer schwimmenden Lagerung 36 gehalten. Diese schwimmende Lagerung 36 gestattet eine Verschiebung des mittleren Rohrabschnitts 26 in axialer Richtung 39. Darüber hinaus ist ein Versatz in radialer Richtung zum Zwecke des Toleranzausgleiches möglich.

Die beiden automatenseitigen Abschnitte 25 und 27 des Versorgungsrohrs 24 sind kugelgelagert ausgebildet, zu welchem Zweck entsprechende Kugellager 33 und 34 vorgesehen sind. Dabei ist der bodenseitige Abschnitt 27 über das Kugellager 34 und der deckenseitige Abschnitt 25 über das Kugellager 33 gelagert.

Der untere, das heißt zweite Abschnitt 27 des Versorgungsrohrs 24 ist in axialer Richtung 39 verschieblich ausgebildet. Er wirkt mit dem Hubzylinder 35 zusammen, mittels dem eine Verschiebebewegung des Abschnittes 27 gestattet ist. Die Fign. 3 und 4 zeigen die entkoppelte Ausgangsposition, dergemäß der untere Abschnitt 27 in seine untere Stellung verbracht ist. Die verkoppelte Stellung ist in den Fign. 5 und 6 gezeigt, dergemäß der untere Abschnitt 27 des Versorgungsrohrs 24 mittels des Hubzylinders 35 in Axialrichtung 39 verfahren ist, und zwar mit Bezug auf die Zeichnungsebene nach den Fign. 5 und 6 nach oben, was in der Konsequenz zu einem Einspannen des mittleren Abschnitts 26 zwischen den beiden automatenseitigen Abschnitten führt, und dies unter Ausbildung fluiddichter Verbindungen zwischen den einzelnen Abschnitten.

Die nach der Erfindung vorgesehene Ankoppelung der korbseitigen Sprüharme 28 an die Spülflottenversorgung befindet sich innerhalb des Spülraums 4 und erfolgt zwischen dem unteren Sprüharm 8, der axial verschieblich ist, und dem oberen Sprüharm 7, der axial fesgelegt ist. Im entkoppelten Zustand befindet sich der untere Sprüharm 8 in abgesenkter Position sowie in den Fign. 3 und 4 gezeigt, so dass sich der mittlere Abschnitt 26 des Versorgungsrohrs 24, das schwimmend am Spülkorb 10 gelagert ist, samt Spülkorb 10 über die Spülraumunterkante der Beschickungsöffnung 5 in den Spülraum einführen lässt.

Der mittlere Abschnitt 26 des Versorgungsrohrs 24 ist mit trichterförmigen Endbereichen ausgerüstet, das heißt mit konischen Erweiterungen 37, die der Positionsfindung, Zentrierung und Abdichtung dienen. Die automatenseitigen Abschnitte 25 und 27 des Versorgungsrohrs 24 sind mit zu den trichterförmigen Enden entsprechend ausgebildeten Kontaktflächen ausgerüstet, so dass im verkoppelten Zustand eine fluiddichte Verbindung entsteht.

Das Ankoppeln oder Verkoppeln selbst erfolgt durch eine Hubbewegung in Axialrichtung 39 des unteren Rohrabschnitts 27 mittels eines Hubzylinders, der beispielsweise druckluftbetrieben ist. Dabei wird der mittlere Abschnitt 26 des Versorgungsrohrs 24 zunächst am unteren Trichter zentriert und angehoben, so dass der obere Trichter sich an der Konusfläche des oberen Rohrabschnitts 25 zentriert und fest andrückt.

Gemäß einer bevorzugten Ausführungsform findet ein elektromotorischer Antrieb der Sprüharme 7, 8 und 28 der Sprüheinrichtung 23 statt. Zu diesem Zweck ist ein Motor 19 vorgesehen, der über eine Antriebswelle 20 an die verdrehbar gelagerten Sprüharme 7, 8 und 28 angeschlossen ist. Dabei ist es bevorzugt, dass die Verbindung der einzelnen Abschnitte 25, 26 und 27 des Versorgungsrohrs 24 kraftübertragend ausgebildet ist, so dass über eine Antrieb allein des unteren zweiten Abschnitts 27 des Versorgungsrohrs 24 ein Antrieb sämtlicher Sprüharme 7, 8 und 28 erreicht ist. Um dies sicherzustellen wird die Reibkraft der Konus-Kontaktflächen genutzt. Diese ist größer als die Rollreibung der Kugellager 33 und 34, so dass bei einer Motoransteuerung die eingeleitete Drehbewegung vom unteren Abschnitt 27 auf den mittleren Abschnitt 26 und von dort aus auf den oberen Abschnitt 25 übertragen wird.

Die Antriebswelle 20 erstreckt sich bevorzugterweise entlang der Drehachse 9, zu welchem Zweck die Pumpenwelle 18 als Hohlwelle ausgebildet ist, durch die hindurch die Antriebswelle 20 zum Antrieb der Sprüharme 7, 8 und 28 geführt ist.

Gemäß einer alternativen Ausgestaltung der Erfindung kann ein Antrieb der Sprüharme 7, 8 und 28 auch von oben, das heißt deckenseitig erfolgen. Gemäß dieser Alternative sind ein Motor 31 und eine Welle 32 vorgesehen, die auf den oberen, das heißt ersten Abschnitt 25 des Versorgungsrohrs 24 und den damit gekoppelten Sprüharm 7 einwirken. Der Motor 19 und die Antriebswelle 20 können im Falle dieser alternativen Ausgestaltungsform entfallen.

Die Sprüheinrichtung 23 dient nicht nur der Beschickung von Spülgut mit Spülflotte, sondern auch der Beschickung des Spülguts mit Trocknungsluft. Zu diesem Zweck ist ein Lufteinlass 30 vorgesehen, der an ein in den Fign. nicht näher dargestelltes Gebläse angeschlossen ist. Über den Lufteinlass 30 kann vom Gebläse geförderte Luft in das Versorgungsrohr 24 eingeleitet werden, von wo aus dann eine Verteilung auf die einzelnen Sprüharme 7, 8 und 28 stattfindet.

Neben den in den Fign. gezeigten Sprüharmen 7, 8 und 28 kann die Sprüheinrichtung 23 auch andere Fluidabgabeeinrichtungen aufweisen, zum Beispiel in Form von Anschlussstutzen, an die in einfacher Weise beispielsweise Schläuche durch Aufstecken angeschlossen werden können.

**Bezugszeichenliste**

| | | | |
|---|---|---|---|
| 1 | Spülautomat | 16 | Motor |
| 2 | Gehäuse | 17 | Pumpenrad |
| 3 | Spülbehälter | 18 | Pumpenwelle |
| 4 | Spülraum | 19 | Motor |
| 5 | Beschickungsöffnung | 20 | Antriebswelle |
| 6 | Spülraumtür | 21 | Leitrad |
| 7 | Sprüharm | 22 | Heizeinrichtung |
| 7a | Sprüharmast | 23 | Sprüheinrichtung |
| 7 b | Sprüharmast | 24 | Versorgungsrohr |
| 8 | Sprüharm | 25 | erster Abschnitt |
| 8a | Sprüharmast | 26 | dritter Abschnitt |
| 8b | Sprüharmast | 27 | zweiter Abschnitt |
| 9 | Drehachse | 28 | Sprüharm |
| 10 | Spülkorb | 29 | Kupplungsstelle |
| 11 | Gestell | 30 | Luftanschluss |
| 12 | Sammeltopf | 31 | Motor |
| 13 | Ablaufstutzen | 32 | Welle |
| 14 | Flächensieb | 33 | Kugellager |
| 14a | Reservoir | 34 | Kugellager |
| 15 | Umwälzpumpe | 35 | Hubzylinder |
| 36 | schwimmende Lagerung | | |
| 37 | konische Erweiterung | | |
| 38 | Anschlussstutzen | | |
| 39 | Axialrichtung | | |

## Patentansprüche

1. Reinigungs- und Desinfektionsautomat insbesondere für medizinische Instrumente und Geräte, mit einem einen Spülraum (4) bereitstellenden Spülbehälter (3) und einer im Spülraum (4) angeordneten Sprüheinrichtung (23), wobei die Sprüheinrichtung (23) eine Mehrzahl von um eine gemeinsame Drehachse (9) verdrehbar ausgebildeten Sprüharmen (7, 8, 28) aufweist, sowie mit einem sich entlang der Drehachse (9) der Sprüharme (7, 8, 28) erstreckenden Versorgungsrohr (24) zur Versorgung der Sprüharme (7, 8, 28) mit Spülflotte, wobei das Versorgungsrohr (24) fluiddicht miteinander verbindbare Abschnitte (25, 26, 27) aufweist,
wobei
zwei der Abschnitte (25, 27), nämlich ein bodenseitig angeordneter Abschnitt (27) und ein deckenseitig angeordneter Abschnitt (25), automatenseitig am Spülbehälter ausgebildet sind und jeweils mit einem Sprüharm (7, 8) gekoppelt sind, und wobei ein zwischen diesen zwei Abschnitten (25, 27) angeordneter mittlerer Abschnitt (26) axial verschieblich gelagert von einem dem Spülraum (4) entnehmbaren Spülkorb (10) aufgenommen ist.

2. Automat nach Anspruch 1,
**dadurch gekennzeichnet,**
**dass** der bodenseitig des Spülraums (4) angeordnete Abschnitt (27) oder der deckenseitig des Spülraums (4) angeordneten Abschnitt (25) axial verschieblich gelagert ist.

3. Automat nach Anspruch 2,
**dadurch gekennzeichnet,**
**dass** der bodenseitig des Spülraums (4) angeordnete Abschnitt (27) und der deckenseitig des Spülraums (4) angeordnete Abschnitt (25) des Versorgungsrohrs (24) jeweils an einen Sprüharm (7, 8) angeschlossen und um die Drehachse (9) der Sprüharme (7, 8, 28) verdrehbar gelagert sind.

4. Automat nach Anspruch 2 oder 3,
**dadurch gekennzeichnet,**
**dass** der axialverschieblich im Spülraum (4) gelagerte Abschnitt (27) des Versorgungsrohrs (24) mit einem Hubzylinder (35) zusammenwirkt.

5. Automat nach einem der vorhergehenden Ansprüche,
**dadurch gekennzeichnet,**
**dass** der spülkorbseitige Abschnitt (26) des Versorgungsrohrs (24) endseitig konisch ausgebildete Erweiterungen (37) aufweist.

6. Automat nach Anspruch 5,
**dadurch gekennzeichnet,**
**dass** die spülraumseitigen Abschnitte (25, 27) des Versorgungsrohrs (24) zu den konisch ausgebildeten Erweiterungen (37) des spülkorbseitigen Abschnitts (26) des Versorgungsrohrs (24) korrespondierend ausgebildete Anschlussstutzen (38) aufweisen.

7. Automat nach einem der vorhergehenden Ansprüche 2 bis 6,
**dadurch gekennzeichnet,**
**dass** die spülraumseitigen Abschnitte (25, 27) des Versorgungsrohrs (24) kugelgelagert sind.

8. Automat nach einem der vorhergehenden Ansprüche,
**dadurch gekennzeichnet,**
**dass** der spülkorbseitige Abschnitt (26) des Versorgungsrohrs (24) an Sprüharme (28) oder andere Fluidabgabeeinrichtungen angeschlossen ist.

9. Automat nach einem der vorhergehenden Ansprüche,
**dadurch gekennzeichnet,**
**dass** der spülkorbseitige Abschnitt (26) des Versorgungsrohrs (24) in einzelne fluiddicht miteinander verbindbare Segmente unterteilt ist.

10. Automat nach einem der vorhergehenden Ansprüche,
**dadurch gekennzeichnet,**
**dass** an das Versorgungsrohr (24) einendseitig eine Umwälzpumpe (15) und anderendseitig ein Gebläse angeschlossen ist.

## Claims

1. Automatic cleaning and disinfection machine, in particular for medical instruments and appliances, comprising a washing container (3) which provides a rinsing chamber (4), and a spray device (23) arranged in the rinsing chamber (4), the spray device (23) having a plurality of spray arms (7, 8, 28) which are rotatable about a common axis of rotation (9), and a supply pipe (24) for supplying rinsing solution to the spray arms (7, 8, 28), which pipe extends along the axis of rotation (9) of the spray arms (7, 8, 28), wherein the supply pipe (24) comprises portions (25, 26, 27) which can be interconnected in a fluid-tight manner, wherein two of the portions (25, 27), specifically a portion (27) arranged at the base and a portion (25) arranged at the top, are formed on the washing container on the machine side and are each coupled to one spray arm (7, 8) respectively, and wherein a central portion (26) that is arranged between these two portions (25, 27) is received in an axially movably mounted manner by a dishwasher rack (10) which is removable from the rinsing chamber (4).

2. Automatic machine according to claim 1,
**characterised in that**
the portion (27) arranged at the base of the rinsing chamber (4) or the portion (25) arranged at the top of the rinsing chamber (4) is mounted in an axially movable manner.

3. Automatic machine according to claim 2,
**characterised in that**
the portion (27) of the supply pipe (24) arranged at the base of the rinsing chamber (4) and the portion (25) of the supply pipe arranged at the top of the rinsing chamber (4) are each connected to one spray arm (7, 8) respectively and are rotatably mounted about the axis of rotation (9) of the spray arms (7, 8, 28).

4. Automatic machine according to either claim 2 or claim 3,
**characterised in that**
the portion (27) of the supply pipe (24) that is mounted in the rinsing chamber (4) in an axially movable manner cooperates with a lifting cylinder (35).

5. Automatic machine according to any of the preceding claims,
**characterised in that**
the portion (26) of the supply pipe (24) on the dishwasher rack side has conical extensions (37) on the ends thereof.

6. Automatic machine according to claim 5,
**characterised in that**
the portions (25, 27) of the supply pipe (24) on the rinsing chamber side comprise connecting pieces (38) which are formed so as to correspond to the conical extensions (37) of the portion (26) of the supply pipe (24) on the dishwasher rack side.

7. Automatic machine according to any of the preceding claims 2 to 6,
**characterised in that**
the portions (25, 27) of the supply pipe (24) on the rinsing chamber (4) side are ball-bearing-mounted.

8. Automatic machine according to any of the preceding claims,
**characterised in that**
the portion (26) of the supply pipe (24) on the dishwasher rack side is connected to spray arms (28) or other fluid discharge devices.

9. Automatic machine according to any of the preceding claims,
**characterised in that**
the portion (26) of the supply pipe (24) on the dishwasher rack side is divided into individual segments which can be interconnected in a fluid-tight manner.

10. Automatic machine according to any of the preceding claims,
**characterised in that**
a circulating pump (15) is connected to the supply pipe (24) at one end and a fan is connected at the other end.

## Revendications

1. Appareil automatique de nettoyage et de désinfection, en particulier pour instruments et appareils médicaux, avec une cuve de lavage (3) fournissant un espace de lavage (4) et avec un dispositif de pulvérisation (23) disposé dans l'espace de lavage (4), dans lequel le dispositif de pulvérisation (23) présente une pluralité de bras de pulvérisation (7, 8, 28) constitués de façon à pouvoir tourner autour d'un axe de rotation (9) commun, ainsi qu'avec un tube d'alimentation (24) s'étendant le long de l'axe de rotation (9) des bras de pulvérisation (7, 8, 28) pour l'alimentation des bras de pulvérisation (7, 8, 28) en eau de rinçage, dans lequel le tube d'alimentation (24) présente des tronçons (25, 26, 27) pouvant être raccordés les uns aux autres de façon étanche aux fluides,
dans lequel deux des tronçons (25, 27), à savoir un tronçon (27) disposé côté fond et un tronçon (25) disposé côté sommet, sont constitués sur la cuve de lavage côté appareil automatique et sont couplés respectivement à un bras de pulvérisation (7, 8), et dans lequel un tronçon (26) central disposé entre ces deux tronçons (25, 27) est, en étant supporté de façon axialement coulissante, logé dans un panier de lavage (10) pouvant être enlevé de l'espace de lavage (4).

2. Appareil automatique selon la revendication 1,
**caractérisé en ce que**
le tronçon (27) disposé côté fond de l'espace de lavage (4) ou le tronçon (25) disposé côté sommet de l'espace de lavage (4) est supporté de façon axialement coulissante.

3. Appareil automatique selon la revendication 2,
**caractérisé en ce que**
le tronçon (27) du tube d'alimentation (24) disposé côté fond de l'espace de lavage (4), et le tronçon (25) du tube d'alimentation disposé côté sommet de l'espace de lavage (4) sont respectivement raccordés à un bras de pulvérisation (7, 8) et sont supportés de façon à tourner autour de l'axe de rotation (9) des bras de pulvérisation (7, 8, 28).

4. Appareil automatique selon la revendication 2 ou 3,
**caractérisé en ce que**
le tronçon (27) du tube d'alimentation (24) supporté de façon axialement coulissante dans l'espace de lavage (4) coopère avec un vérin de levage (35).

5. Appareil automatique selon l'une des revendications précédentes,
**caractérisé en ce que**
le tronçon (26) du tube d'alimentation (24) côté panier de lavage présente, côté extrémité, des élargissements (37) constitués de façon conique.

6. Appareil automatique selon la revendication 5,
**caractérisé en ce que**
les tronçons (25, 27) du tube d'alimentation (24) côté espace de lavage présentent des tubulures de raccordement (38) constituées d'une façon correspondante aux élargissements (37) constitués de façon conique du tronçon (26) du tube d'alimentation (24) côté panier de lavage.

7. Appareil automatique selon l'une des revendications précédentes 2 à 6,
**caractérisé en ce que**
les tronçons (25, 27) du tube d'alimentation (24) côté espace de lavage (4) sont montés sur des roulements.

8. Appareil automatique selon l'une des revendications précédentes,
**caractérisé en ce que**
le tronçon (26) du tube d'alimentation (24) côté panier de lavage est raccordé à des bras de pulvérisation (28) ou à d'autres dispositifs de distribution de fluides.

9. Appareil automatique selon l'une des revendications précédentes,
**caractérisé en ce que**
le tronçon (26) du tube d'alimentation (24) côté panier de lavage est divisé en différents segments pouvant être raccordés les uns aux autres de façon étanche aux fluides.

10. Appareil automatique selon l'une des revendications précédentes,
**caractérisé en ce qu'**une pompe de circulation (15) est raccordée au tube d'alimentation (24) à une extrémité et un ventilateur est raccordé à l'autre extrémité.
